# EUROPEAN PATENT APPLICATION

(11) **EP 4 406 967 A1**
(43) Date of publication of application: **31.07.2024**
(21) Application number: 22871531.4
(22) Date of filing: 30.06.2022
(51) Int. Cl.: C07K 14/72, C07K 19/00, G01N 33/68

(54) **RECOMBINANT THYROID STIMULATING HORMONE RECEPTOR PROTEIN, PREPARATION METHOD THEREFOR AND APPLICATION THEREOF**

(30) Priority: 22.09.2021 CN 202111107448
(71) Applicant: Xiamen Innobiomax Biotechnology Co., Ltd., Xiamen, Fujian 361000 (CN); Xiamen Innodx Biotech Co., Ltd, Xiamen, Fujian 361000 (CN)
(72) Inventor: WANG, Haihong, Xiamen, Fujian 361000 (CN); KE, Qishen, Xiamen, Fujian 361000 (CN); ZOU, Mengwen, Xiamen, Fujian 361000 (CN); ZHUANG, Cuizhen, Xiamen, Fujian 361000 (CN); LI, Lihua, Xiamen, Fujian 361000 (CN); LI, Huiping, Xiamen, Fujian 361000 (CN); CHEN, Weibin, Xiamen, Fujian 361000 (CN); KE, Shaojun, Xiamen, Fujian 361000 (CN); ZHANG, Yifei, Xiamen, Fujian 361000 (CN); LIN, Qiumin, Xiamen, Fujian 361000 (CN); SONG, Liuwei, Xiamen, Fujian 361000 (CN); XIONG, Junhui, Xiamen, Fujian 361000 (CN)
(74) Representative: Brann AB
(86) International application number: PCT/CN2022/102890
(87) International publication number: WO 2023/045470

(57) **Abstract**

The present invention provides a recombinant thyroid stimulating hormone receptor protein, a preparation method therefor and an application thereof. The recombinant thyroid stimulating hormone receptor protein comprises a TSHR protein extracellular segment and a TSHR protein intracellular segment. The C-terminus of the TSHR protein extracellular segment is linked to the N-terminus of the TSHR protein intracellular segment. The protein achieves extracellular soluble expression, and is high in purity, high in activity and good in stability.

## Description

### Technical Field

The present invention relates to a recombinant thyroid-stimulating hormone receptor protein, preparation method and application thereof, and belongs to the technical field of preparation of thyroid-stimulating hormone receptor protein.

### Background Art

Thyroid-stimulating hormone receptor (TSHR) is a G protein-coupled receptor present on the membrane of thyroid follicular cells. Under normal physiological conditions, TSHR regulates the growth, differentiation and function of thyroid follicular cells by binding with thyroid-stimulating hormone (TSH), and through the adenylate cyclase (AC-cAMP) pathway and the phospholipase C-diacylglycerol and phosphoinositide pathway. TSHR not only mediates the physiological effects of TSH, but also is an important autoantigen. Its corresponding autoantibody, thyroid-stimulating hormone receptor antibody (TSHRAb, TRAb), plays an important role in the occurrence of autoimmune thyroid disease (AITD). TRAb mediates the occurrence and development of autoimmune thyroid disease, and is considered as an important indicator for the detection of patients with autoimmune thyroid disease.

At present, there are two main types of methods for TRAb detection, one is the competition method, and the other is the double-antigen sandwich method. In the competition method, the patient's own TRAb is capable of competing with the labeled recombinant human stimulatory monoclonal autoantibody M22 for binding to TSHR thereby quantifying the TSHR, and the method has higher sensitivity and better detection stability than traditional method in which TRAb and TSH compete for binding to TSHR, and it is also the main TRAb detection method at present. The other is the double-antigen sandwich method, which uses more than two TSHR antigens to perform sandwich detection of TRAb, this type of detection method has not been widely used clinically at present. No matter which of the above detection methods is used, it is necessary to achieve the quantification of TRAb through the binding of TRAb and TSHR. Therefore, as a key raw material for TRAb detection, it is crucial for TSHR to meet the performance requirements of clinical detection and the needs of industrial production.

Since the value of TRAb in AITD disease was discovered, researchers and related technicians in the field have never stopped studying methods for preparing TSHR proteins. At present, the preparation methods of TSHR protein mainly include natural extraction methods and genetic engineering methods.

The natural extraction method is to extract TSHR from other species (mainly pigs) to replace human TSHR. However, since TSHR is at very low level in thyroid follicular cells, TSHR is a seven-transmembrane protein with low protein solubility (high concentration of surfactant must be added), and TSHR is very unstable, the difficulty of natural extraction increases significantly. The purity and content of natural antigens obtained so far are relatively low, the process is relatively complex and cumbersome, and there are obvious differences between batches. In addition, the high concentration of surfactant in the extract makes the antigens unable to be used directly. All these factors limit the development of subsequent research work.

Since Nagayama successfully cloned human TSHR cDNA in 1989, many researchers have tried a variety of genetic engineering methods to recombinantly express TSHR on different expression systems. Loosfelt, Harfst and others tried to use *Escherichia coli* to express the full-length and extracellular segments of TSHR, but they were unable to achieve soluble expression. The protein existed in the form of inclusion bodies and required urea denaturation and electroelution to obtain protein with higher purity, but the purification with urea and electroelution destroyed the structure of the protein, so that the protein was unable to bind to TSH and TRAb and could not be used in subsequent research and applications. Harfst in 1992, Huang in 1993, and Fowler et al. in 1995 tried to use baculovirus-insect expression system to express the full-length TSHR. Although high levels of mRNA were detected, TSHR was not successfully expressed. These proteins were either insoluble in water, or denatured, or lack of glycosylation modification. In 1995, Chazenbalk and Rapoport achieved the expression of the extracellular segment by introducing an early promoter, but the expression level was still low. For a long time, researchers believed that the baculovirus system was not suitable for recombinant expression of TSHR. Wedlock et al. attempted to recombinantly express the full-length and extracellular segments of TSHR in the yeast expression system by introducing the leader sequence STE2 and α factor, but all ended in failure.

So far, many researchers have used mammalian cells to express TSHR, which is considered the most effective method to recombinantly express full-length TSHR. However, despite this, its expression level is still very low, and extracellular secretion expression has not been achieved. Since the protein is expressed within the cell, it is necessary to add multiple steps such as cell collection, cell washing, cell disruption, centrifugation of the disrupted product to collect the supernatant, etc., in order to further purify and obtain the recombinant TSHR protein. Since TSHR itself is unstable, each additional step will increase the uncertainty of the purified product and influence the stability of application effects of the product.

In summary, the current preparation methods of TSHR protein have various problems such as low expression level, low purity, low yield, complex and cumbersome extraction procedures, poor stability, large batch-to-batch differences in extraction products, high surfactant concentration in extraction products, and inability to extracellular soluble expression, and thus cannot be directly used in TRAb detection.

### Contents of the Invention

The present invention provides a recombinant thyroid-stimulating hormone receptor protein, preparation method thereof and use thereof, which can effectively solve the above problems.

The present invention is implemented as follows:
A recombinant thyroid-stimulating hormone receptor protein, comprising an extracellular segment of TSHR protein and an intracellular segment of TSHR protein, the C-terminus of the extracellular segment of TSHR protein is connected to the N-terminus of the intracellular segment of TSHR protein.

As a further improvement, the amino acid sequence of the extracellular segment of TSHR protein is set forth in SEQ ID NO: 12, and the amino acid sequence of the intracellular segment of TSHR protein is set forth in SEQ ID NO: 13.

As a further improvement, the N-terminus is further connected to an exogenous secretion signal peptide.

As a further improvement, the C-terminus of the intracellular segment of TSHR protein is further connected to a purification tag sequence.

A method for preparing the above recombinant thyroid-stimulating hormone receptor protein, comprises the following steps:
S1, constructing a recombinant plasmid, in which the recombinant plasmid comprises a gene fragment of the extracellular segment of TSHR protein and a gene fragment of the intracellular segment of TSHR protein; the C-terminus of the extracellular segment of TSHR protein is connected to the N-terminus of the intracellular segment of TSHR protein;
S2, transferring the recombinant plasmid into an eukaryotic expression system for expression and identification of a target protein;
S3, purifying the target protein.

As a further improvement, the method of constructing the recombinant plasmid comprises: using an overlapping PCR method to ligate the gene fragment of the extracellular segment of TSHR protein and the gene fragment of the intracellular segment of TSHR protein to form a target gene fragment, and then using a Gibson cloning method to construct the target gene onto a plasmid vector.

As a further improvement, the method of constructing the recombinant plasmid comprises chemically synthesizing a target gene fragment comprising the gene fragment of the extracellular segment of TSHR protein and the gene fragment of the intracellular segment TSHR protein, and then constructing the target gene fragment onto a plasmid vector.

As a further improvement, the eukaryotic expression system is selected from the group consisting of baculovirus-insect expression system, CHO cell expression system, 293 cell expression system, or yeast expression system.

A use of the above recombinant thyroid-stimulating hormone receptor protein in the detection of thyroid-stimulating hormone receptor antibody.

A kit for detecting thyroid-stimulating hormone receptor antibody, comprises the above-mentioned recombinant thyroid-stimulating hormone receptor protein.

The beneficial effects of the present invention are:
In the present invention, the extracellular segment and the intracellular segment of TSHR are expressed in series to realize the structural support of the intracellular segment protein to the extracellular segment protein, so as to increase the stability of the extracellular segment protein, thereby improving the activity of the protein, which can be used for the development of TRAb detection reagent; in addition, the deletion of transmembrane segment can increase extracellular soluble expression and simplify the purification process. The protein preparation method is simple with high yield.

### Brief Description of the Drawings

In order to more clearly illustrate the technical solutions of the embodiments of the present invention, the drawings needed to be used in the embodiments will be briefly introduced below. It should be understood that the following drawings only show certain examples of the present invention and therefore should not be regarded as a limitation of the scope. For those of ordinary skill in the art, other relevant drawings can be obtained based on these drawings without exerting creative efforts.
FIG. 1 shows the identification electropherogram of the T1, T2, and T3 fragments recovered in Example 1 of the present invention. Lane 1: T1 fragment, Lane 2: T2 fragment, Lane 3: T3 fragment, Lane 4: Nucleic acid Marker.
FIG. 2 shows the identification electropherogram of the T4 fragment recovered in Example 1 of the present invention. Lane 1: T4 fragment, Lane 2: Nucleic acid Marker.
FIG. 3 shows the identification electropherogram of the T5 fragment recovered in Example 1 of the present invention. Lane 1: T5 fragment, Lane 2: Nucleic acid Marker.
FIG. 4 shows the cell morphology of H5 cells before infection with baculovirus provided in Example 1 of the present invention.
FIG. 5 shows the cell morphology of H5 cells after infection with baculovirus provided in Example 1 of the present invention.
FIG. 6 shows the SDS-PAGE and corresponding WB results of the target protein provided in Example 1 of the present invention. Lane 1: Expression supernatant, Lane 2: Purified target protein, Lane 3: Non-related protein fused with human FC tag, Lane 4: Protein Marker.
FIG. 7 shows the quantitative curve of the prototype reagent provided in Example 2 of the present invention.
FIG. 8 shows the correlation between a commercial reagent and the prototype reagent constructed with the recombinant protein that is provided in Example 2 of the present invention.

### EXAMPLES

In order to make the purpose, technical solutions and advantages of the embodiments of the present invention clearer, the technical solutions in the embodiments of the present invention will be clearly and completely described below in conjunction with the drawings in the embodiments of the present invention. Obviously, the described embodiments are a part of embodiments of the present invention, but not all of them. Based on the embodiments of the present invention, all other embodiments obtained by those of ordinary skill in the art without creative efforts fall within the scope of protection of the present invention. Accordingly, the following detailed description of embodiments of the present invention provided in the appended drawings is not intended to limit the scope of the claimed invention, but rather to represent selected embodiments of the present invention. Based on the embodiments of the present invention, all other embodiments obtained by those of ordinary skill in the art without creative efforts fall within the scope of protection of the present invention.

In the description of the present invention, the terms "first" and "second" are used for descriptive purposes only and cannot be understood as indicating or implying relative importance or implicitly indicating the number of indicated technical features. Therefore, features defined as "first" and "second" may explicitly or implicitly include one or more of these features. In the description of the present invention, "plurality" means two or more than two, unless otherwise explicitly and specifically limited.

The present invention provides a recombinant thyroid-stimulating hormone receptor protein, which comprises an extracellular segment of TSHR protein and an intracellular segment of TSHR protein, in which the C-terminus of the extracellular segment of TSHR protein is connected to the N-terminus of the intracellular segment of TSHR protein. The TSHR protein can be derived from human, or other animals that are closely related to human, such as pig, cattle, and orangutan; or the TSHR protein is a TSHR protein derived from the above source with individual amino acid mutations, in which the individual amino acid mutations do not affect soluble expression and maintenance of activity.

Preferably, the amino acid sequence of the extracellular segment of TSHR protein is set forth in SEQ ID NO: 12; the amino acid sequence of the intracellular segment of TSHR protein is set forth in SEQ ID NO: 13. More preferably, the extracellular segment of TSHR protein has an amino acid sequence corresponding to the 64-1248 bp fragment of the human full-length TSHR DNA; and the intracellular segment of TSHR protein has an amino acid sequence corresponding to the 2035-2292 bp fragment of the human full-length TSHR DNA.

The N-terminus of the recombinant thyroid-stimulating hormone receptor protein may be further connected to an exogenous secretion signal peptide, which is beneficial to the soluble expression of the protein. If the selected expression vector has its own secretion signal peptide, the N-terminus of the recombinant thyroid-stimulating hormone receptor protein does not need to be connected to an exogenous secretion signal peptide.

Preferably, the C-terminus of the intracellular segment of TSHR protein may by further connected to a purification tag sequence, and the purification tag sequence includes but not limited to human recombinant IgG1 FC, 6×His, and GST tag sequence, which firstly increases soluble expression, and secondly effectively separate the target fragment from the expression supernatant for affinity chromatography tag, thereby improving the purity of the protein.

A method for preparing the above recombinant thyroid-stimulating hormone receptor protein, comprises the following steps:
S1, constructing a recombinant plasmid, in which the recombinant plasmid comprises a gene fragment of an extracellular segment of TSHR protein and a gene fragment of an intracellular segment of TSHR protein; the C-terminus of the extracellular segment of TSHR protein is connected to the N-terminus of the intracellular segment of TSHR protein. Preferably, the recombinant plasmid further comprises a gene fragment of a purification tag sequence, and the purification tag sequence is connected to the C terminus of the intracellular segment of TSHR protein. In this method, the extracellular and intracellular segments of TSHR are expressed in series to achieve structural support of the intracellular segment protein to the extracellular segment protein, thereby increasing the stability of the extracellular segment protein, improving the activity of the protein, meanwhile the deletion of transmembrane segment can increase the soluble expression and simplify the purification process. The deleted transmembrane segment is the seven-transmembrane domain, comprising the intramembrane part, and also the extramembrane part in the middle of the transmembrane segment.
S2, transferring the recombinant plasmid into an eukaryotic expression system for expression and identification of the target protein.
S3, purifying the target protein, in which the purification method is a conventional protein purification technology, which will not be described in detail here.

The method for constructing the recombinant plasmid comprises: using an overlapping PCR method to connect the gene fragment of the extracellular segment of TSHR protein and the gene fragment of the intracellular segment of TSHR protein to form a target gene fragment, and then using the Gibson cloning method to construct the target gene fragment onto a plasmid vector. Preferably, the target gene fragment further comprises a gene fragment of a purification tag sequence.

Alternatively, the method of constructing the recombinant plasmid comprises: chemically synthesizing a target gene fragment comprising a gene fragment of the extracellular segment of TSHR protein and a gene fragment of the intracellular segment TSHR protein, and then constructing the target gene fragment onto a plasmid vector. Preferably, the target gene fragment further comprises a gene fragment of a purification tag sequence. The order in which the synthesis is carried out can be individual synthesis of each fragment, combined synthesis of multiple fragments, or direct synthesis into an expression vector in one step.

As a further improvement, the eukaryotic expression system may include but not limited to baculovirus-insect expression system, CHO cell expression system, 293 cell expression system or yeast expression system. More preferably, the eukaryotic expression system is a baculovirus-insect expression system where the expressed protein has the highest stability and the highest protein activity.

A use of the above recombinant thyroid-stimulating hormone receptor protein in the detection of thyroid-stimulating hormone receptor antibody.

A kit for detecting thyroid-stimulating hormone receptor antibody comprises the above-mentioned recombinant thyroid-stimulating hormone receptor protein.

The reagents and instruments used in the examples of the present invention were as follows: Reagents used for PCR (including phusion polymerase, buffer, dNTP) were purchased from TransGen Biotech Co., Ltd.; T4 ligase, DNA Marker, protein Marker, BamHI and other restriction endonucleases, and alkaline phosphatase substrate (NBT/BCIP) were purchased from Thermo Fisher Scientific; Gibson assembly solution was prepared by the applicant; agarose was purchased from Biowest; LipofectamineTM2000 was purchased from Thermo Fisher; the primary antibody used for WB was produced by our company, and the secondary antibody was purchased from KPL; DNA purification and recovery kits and plasmid extraction kits were purchased from TIANGEN; ampicillin was purchased from BBI; Linearized Baculovirus DNA, ESF921 cell culture medium were purchased from Expression Systems, SIMHF cell culture medium was purchased from Sino Biological Inc., fetal bovine serum was purchased from PAN Biotech; Ni medium was purchased from GE Company of the United States; trypan blue was purchased from Sigma. The stimulatory TRAb antibody was purchased from RSR Company, the magnetic particles were purchased from Life Science AS, and the acridinium ester was from Suzhou Zhongsheng Chemical Co., Ltd.

The ordinary PCR instrument in plate format was purchased from Biometra, the horizontal electrophoresis instrument RDY-SP1Z was purchased from Shanghai Shanfu, the constant temperature incubator was purchased from Shanghai Zhicheng, the constant temperature shaker ZWYR-D2401 was purchased from Shanghai Zhicheng, the ultra-clean workbench BSC-1000IIA2 was purchased from Shanghai Boxun Industrial Co., Ltd., the centrifuge was purchased from Thermo Scientific, the inverted microscope was purchased from Guangzhou Yuexian, the vertical electrophoresis instrument was purchased from Bio Rad, the transfer instrument was purchased from Bio Rad, the gel imager 805 was purchased from Shanghai Shanfu, and the cell counter was purchased from ALIT, and the MK10 dry-type thermostat was purchased from Hangzhou Aosheng. The fully automatic chemiluminescence instrument Caris200 was purchased from Xiamen Youmaike Medical Instrument Co., Ltd.

The cell lines, strains and plasmids used in the examples of the present invention were as follows:
Human TSHR (hTSHR, human thyroid-stimulating hormone receptor) full-length gene and PCR primers were synthesized by General biol; plasmid pAcGP67-B was a commercial vector; cells SF9 and H5 were commercial cells; competent cells Trans5α were purchased from Kangti Life Technologies Corporation.

### Example 1: Preparation method of recombinant thyroid-stimulating hormone receptor protein

### Clone Design

On the basis of the cDNA sequence of human TSHR (hTSHR) (SEQ ID NO: 1) and the cDNA sequence of human antibody IgG1 (SEQ ID NO: 2), primers for amplifying hTSHR 64-1248bp (extracellular segment) and 2035-2292bp (intracellular segment) were designed using primer5.0: upstream primer sequence PF1 as set forth in SEQ ID NO: 3; upstream primer sequence PF2 as set forth in SEQ ID NO: 4; upstream primer sequence PF3 as set forth in SEQ ID NO: 5; downstream primer sequence PR1 as set forth in SEQ IDNO: 6; downstream primer sequence PR2 as set forth in SEQ IDNO: 7; downstream primer sequence PR3 as set forth in SEQ IDNO: 8. BamHI and NotI were used as restriction sites. The above sequences and primers were all synthesized by General biol.

The codon-optimized nucleotide sequence of hTSHR 64-1248 bp was set forth in SEQ ID NO: 9; the codon-optimized nucleotide sequence of hTSHR 2035-2292 bp was set forth in SEQ ID NO: 10.

The amino acid sequence of human TSHR (hTSHR) was set forth in SEQ ID NO: 11.

The amino acid sequence of the extracellular segment of hTSHR was set forth in SEQ ID NO: 12; the amino acid sequence of the intracellular segment of hTSHR was set forth in SEQ ID NO: 13.

### Construction of recombinant plasmid:

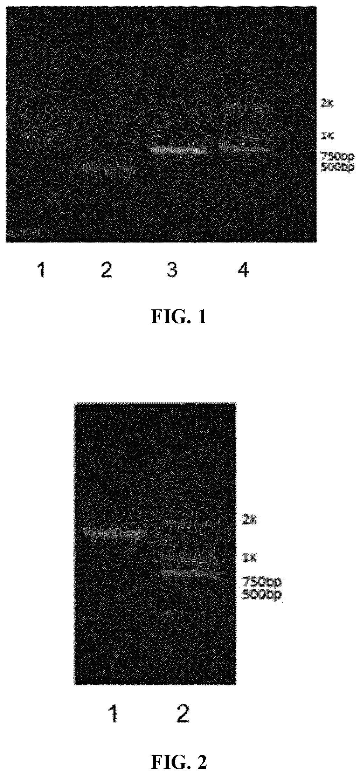

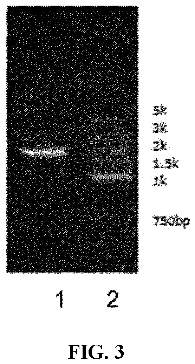

1. QuickCut BamHI and QuickCut NotI were used for double-digestion of pAcGP67-B vector (this vector had its own secretion signal peptide, and no additional signal peptide was required). After appropriate time in a 37°C water bath, the digested vector was subjected to 1% agarose gel electrophoresis, and purified by a gel recovery kit to obtain a linear vector.
2. By using hTSHR DNA as a template, the DNA sequence of hTSHR 64-1248 (T1) was amplified with PF1 and PR1 as primers, and the DNA sequence of hTSHR 2035-2292 (T2) was amplified with PF2 and PR2 as primers; and the human antibody constant region DNA sequence (T3) was amplified by using human antibody IgG1 DNA as a template, and PF3 and PR3 as primers. After the PCR amplification, the DNA fragments T1 (positions 64-1248) and T2 (positions 2035-2292), and the human antibody constant region sequence T3 were subjected to 1% agarose gel electrophoresis, purified, recovered and identified by a gel recovery kit. The results were shown in FIG. 1.
3. By using the overlapping PCR method, using PF1 and PR2 as primers, and using the PCR recovery products T1 and T2 as templates, the purified and recovered DNA fragments of positions 64-1248/2035-2292 were subjected to PCR splicing to obtain a PCR product (T4). The PCR product T4 was subjected to 1% agarose gel electrophoresis, purified, recovered and identified by a gel recovery kit; and the identification results were shown in FIG. 2.
4. By using the overlapping PCR method, using PF1 and PR3 as primers, and using the PCR recovery products T3 and T4 as templates, the 64-1248bp/2035-2292bp gene sequence was subjected to PCR splicing with the human antibody constant region sequence to obtain a PCR product (T5). The PCR product T5 was subjected to 1% agarose gel electrophoresis, purified, recovered and identified by using a gel recovery kit; and the identification results were shown in FIG. 3. The target fragment was thereby obtained.
5. An amount of 2µl of the purified and recovered linear vector and target fragment was taken, and diluted with sterile water to a concentration of 0.1ug/µL for later use.
6. The above-mentioned recovered and purified target fragment and linear vector were assembled by using Gibson assembly solution (5.6mU/µL T5 exonuclease; 33.3mU/µL phusion polymerase; 5.3U/µl Taq ligase; 0.27mM dNTP), with the molar ratio of the target fragment to the linear vector of 4:1, and the system was as follows: linear vector fragment: 1 µL; DNA fragment comprising hTSHR position 64-1248/2035-2292 fragment and human antibody constant region connected in series (i.e., target fragment): 4 µL; Gibson assembly solution: 5 µL; the system was vortexed to mix, and then was placed in a 50°C water bath for 1 hour. The ligation system was then transformed into *Escherichia coli* competent cells Trans5α (purchased from Kangti Life Technologies Corporation). During transformation, positive and negative controls were set. The positive control was a plasmid that had not been digested, and used for detecting the transformation efficiency; the negative control was an empty *E. coli* competent cell, and used for detecting whether the competent cells were contaminated. The transformation product was spread into LB solid medium containing ampicillin (100 µL/mL) and cultured at 37°C overnight. A plurality of single colonies were randomly picked from the LB plate containing the recombinant plasmid, and used as templates to perform PCR amplification reaction using universal primers, and 1% agarose gel electrophoresis was carried out to detect whether there was a target band. At the same time, the plurality of single colonies as picked were separately shaken and subjected to plasmid extraction according to the instructions of the plasmid extraction kit, and then the obtained plasmids were subjected to double-digestion using corresponding restriction endonuclease, and the digested product was subjected to electrophoresis.
7. The samples that were confirmed as positive in both bacterial liquid PCR and double-digestion were subjected to sequencing by a third-party, to confirm the correctness of plasmid construction.
8. Preparation of high-purity recombinant plasmid

### TIANGEN's plasmid extraction kit was used for operation:

1) For high-copy plasmids, 5 mL of overnight LB culture was used, the bacterial cells were collected, centrifuged at 8000 rpm for 5 minutes, and the supernatant was discarded;
2) 250µl of P1 (RNaseA had been already added) was added to resuspend the bacterial cells, and mixed evenly by pipetting;
3) 250µl of P2 was added, and mixed gently by inverting 7 to 8 times (no vortexing);
4) 350µl of P3 was added, and immediately mixed gently by inverting 7 to 8 times (no vortexing);
5) Centrifugation was carried out at 12,000 rpm at room temperature for 10 minutes;
6) The centrifugation supernatant was transferred to adsorption column, centrifuged at 12,000 rpm for 1 min, and the centrifugate was discarded;
7) 600µl of PW rinse solution was added to the column, centrifuged at 12000rpm for 1 minute, the centrifugate was discarded, and the rinse operation was repeated once;
8) The column was centrifuged at 12,000 rpm for 1 minute, and transferred to a new centrifuge tube, placed in a dry-type constant-temperature heater, and dried at 50°C for 10 minutes;
9) 50µl of ddH₂O was added to the adsorption column, allowed to stand for 2 minutes, and centrifuged at 12000 rpm for 2 minutes;
10) 2µl of the centrifugate was taken and diluted 50 times with sterile water for later use, and the concentration of the vector DNA was calculated by using a DU730 spectrophotometer.

### Expression and identification of target protein (baculovirus-insect expression system)

### 1. Baculovirus preparation:

(1) Before transfection, SF9 cells (insect cells) in logarithmic growth phase were inoculated into a 24-well culture plate, with 2.5 to 2.8×10^5 cells per well, and with 500ul of serum-free SIMHF medium. The incubation was carried in a 27 °C humidified incubator for 30 minutes.
(2) Preparation of liposome/DNA complex
   Solution 1: 1µl of the purified hTSHR/pAcGP67-B recombinant plasmid and 1µl of Linearized Baculovirus DNA were taken and diluted in 50µl of Medium.
   Solution 2: 2µl of Lipofectamine TM 2000 was diluted in 50µl of Medium.
      The above Solution 1 and Solution 2 were mixed evenly to form the liposome/DNA complex, which was placed at room temperature and incubated for 30 minutes.
(3) Transfection: The original culture medium of the 24-well plate was pipetted and discarded, then the plate was rinsed with 1ml of serum-free SIMHF medium, and finally replenished with 500µl of serum-free SIMHF medium; the prepared liposome/DNA complex was slowly added to the 24-well culture plate, gently mixed, and cultured in a humidified incubator at 27°C overnight. On the next day, 1 ml of SIMHF medium containing FBS was added to the 24-well plate, and continuously cultured for 3 days, and the resultant virus was of the P0 generation. The empty plasmid pAcGP67-B was transfected using the same method as a negative control.
(4) Amplification of P1 generation virus: After transfection for 72 hours, the cells in the 24-well plate were transferred to a 10cm plate with a cell density of 1×10^6 cells/mL and placed in a humidified incubator at 27°C for 72 hours.
(5) Amplification of P2 generation virus: After adherent culture for 72 hours, the adherent cells in the 10cm plate were resuspended by an electric pipette, the culture medium was enriched in a sterile tube, and 2 ml of the culture medium was transferred to a 100ml Erlenmeyer flask containing 50 ml of cells with a density of 1×10^6 cells/mL, and placed in a constant temperature shaker at 27°C to perform suspension culture for 96 hours, and the resultant virus was of the P2 generation. The rest enriched culture medium (P1 generation virus) was stored in the dark at 4°C.
(6) Amplification of P3 generation virus: 2ml of P2 generation culture medium was transferred to a 100ml Erlenmeyer flask containing 50ml of cells with a density of 1×10^6 cells/mL. After suspension culture for 96 hours, 20µl of the culture medium was taken and added with 20µl of trypan blue for staining, then a cell counter was used to monitor the cytopathic effect, the culture medium was enriched in a sterile tube, and stored in the dark at 4°C for protein expression.

### 2. Expression of target protein in High-Five (H5) cells (insect cells):

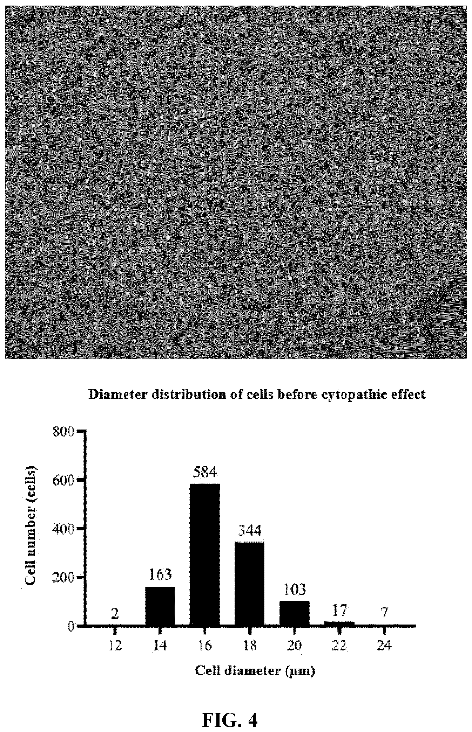

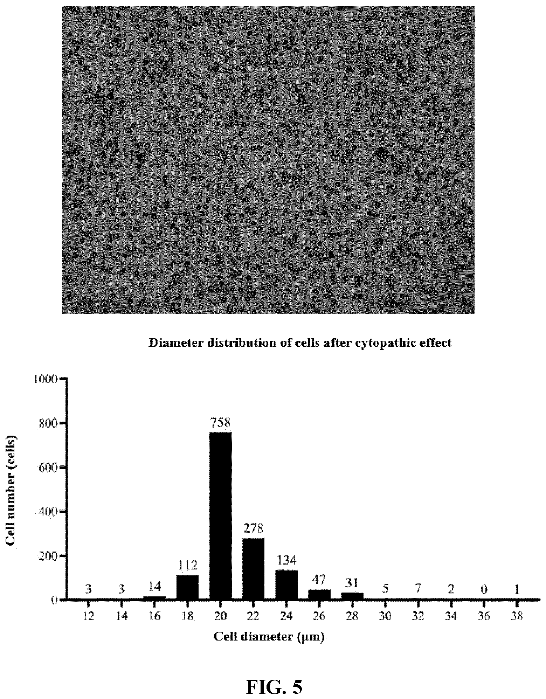

H5 cells were diluted to have a density of 3.0×10^6 cells/mL, with a total volume of 500ml, a cell viability greater than 98%, and a cell diameter of 17-17.5µm, then added with 5ml of P3 generation virus containing the target gene and placed in a constant temperature shaker at 27°C for culture; after 72 hours, 20µl of the culture medium was taken and added with 20µl of trypan blue for staining, and a cell counter was used to monitor the cytopathic effect. The results before and after the cytopathic effect were shown in FIGS. 4 and 5, in which the diameter distribution of normal cells before the expression at 25x magnification was calculated using Countstar (purchased from ALIT). The cells were effectively infected with the virus, and the cell diameter became significantly larger. After the cell viability dropped to a certain level, the culture supernatant was collected for activity determination as well as protein purification and identification.

### 3. Activity determination of expression product in culture supernatant:

The cell supernatant after expression was detected by acridinium ester chemiluminescent double-antibody sandwich detection method that was constructed using one TSHR C-terminal mouse antibody 10F8 and one human stimulatory TRAb. It was found that the expression product had high activity. The specific detection method was as follows:

### 3.1 Magnetic beads coating of 10F8

(1) The magnetic particles and EDC were equilibrated to room temperature, and the magnetic particles with a concentration of 4mg/ml was added with a certain concentration of activator EDC, and allowed to stand at room temperature for activation for 30 minutes.
(2) The activated magnetic particles were subjected to removal of supernatant by using a magnetic collection device, and added into a MES buffer solution for resuspension.
(3) A certain amount of anti-TSHR antibody 10F8 was added, mixed well, and reacted at room temperature for 3 hours.
(4) After magnetic collection by a magnetic stand, the supernatant was discarded, and an equal volume of a phosphate buffer containing a certain amount of protein was added, and reacted at room temperature for 3 hours.
(5) After magnetic collection by a magnetic collection device, the supernatant was discarded, and an equal volume of a cleaning solution was added for repeat washing 4 times.
(6) The cleaned magnetic particles were stored in a magnetic bead diluent and stored at 2 to 8°C.

### 3.2 Acridinium ester labeling of human stimulatory TRAb

(1) Acridinium ester was added to phosphate buffer containing the stimulatory TRAb antibody and mixed well, then reacted at room temperature for 30 minutes in the dark.
(2) A stopping buffer was added, mixed well, and reacted at room temperature in the dark for 30 minutes.
(3) After the acridinium ester label was removed by dialysis, an equal volume of glycerin was added, mixed well, and stored in the dark at -20°C.

### 3.3 Activity detection of expression product in supernatant

The H5 cell expression supernatant was diluted into a series of samples at a certain ratio. The recombinant hTSHR in the sample reacted with the 10F8-coated magnetic particle for 15 minutes to form a 10F8-coated magnetic particle-recombinant hTSHR complex; after magnetic separation and washing, other substances that were not bound to magnetic particles were removed. Next, the acridinium ester-labeled stimulatory TRAb was added and reacted with the recombinant hTSHR in the sample for 10 minutes to form a "10F8-coated magnetic particle-recombinant hTSHR-acridinium ester-labeled stimulatory TRAb" complex. After magnetic separation and washing, other substances that were not bound to the magnetic particles are removed. Finally, after adding pre-trigger solution and trigger solution, the complex could emit a light signal with an intensity detectable by a luminometer, expressed as a relative luminescence value (RLU). The mean light signal intensity was proportional to the amount of recombinant hTSHR in the sample. The detection results of gradient dilution samples of H5 expression supernatant showed that the expression product in the cell supernatant had high activity (Table 1).

**Table 1**

| Dilution factor | RLU (MEAN) |
|---|---|
| Original times | 876212 |
| 1:25 | 148556 |
| 1:50 | 112467 |
| 1:100 | 58663 |
| 1:200 | 27148 |
| Diluent | 4526 |

### Purification and identification of target protein:

### 4.1 Purification of target protein

The enriched H5 cell culture medium was centrifuged at 8000rpm for 10 minutes. The supernatant was dialyzed into 20mM PBS buffer. The supernatant after dialysis was with a pH of about 7.2, and subjected to protein purification by directly using a Protein A column, and gradient elution with citric acid, the elution peak of citric acid with a concentration of 70% was collected, and the target protein was harvested after dialysis.

### 4.2 WB identification of cell supernatant and purified protein activity

(1) Sample preparation: 50µl of H5 cell culture supernatant, 50µl of purified protein, and 50µl of irrelevant protein fused with human FC were taken, added with 10µl of 6×SDS gel loading buffer, mixed, boiled in boiling water for 10 minutes, and centrifuged at 10,000g for 10 minutes;
(2) Electrophoresis: The above protein samples were loaded and subjected to 12% SDS-PAGE at a constant voltage of 80V;
(3) Transfer to membrane: The protein was transferred to nitrocellulose membrane for 45 min at 35mA/gel;
(4) Blocking: Shaking was slowly carried out in 5% skimmed milk powder blocking solution for 1 hour;
(5) Primary antibody reaction: The hTSHR C-terminal antibody 10F8 was diluted to 10ug/ml with 5% skim milk powder, with a total volume of 10ml, the above-mentioned transferred nitrocellulose membrane was placed into the prepared primary antibody, and reacted for 30 minutes;
(6) Washing: The above nitrocellulose membrane was washed with 11×TNT for 5 times, 3 to 5 minutes each time;
(7) Secondary antibody incubation: KPL GAM-AP was diluted with 5% skimmed milk powder at 1:5K with a total volume of 10ml, the above-mentioned transferred nitrocellulose membrane was placed into the prepared primary antibody and reacted for 30 minutes;
(8) Washing: The above nitrocellulose membrane was washed with 1×TNT for 5 times, 3 to 5 minutes each time;
(9) Color development and termination: 10 ml of diluted BCIP/NBT chromogenic solution was added, the chromogenic reaction was carried out in the dark for 15 minutes, the chromogenic solution was discarded, followed by washing once with deionized water, and after photographing, it was stored in the dark.

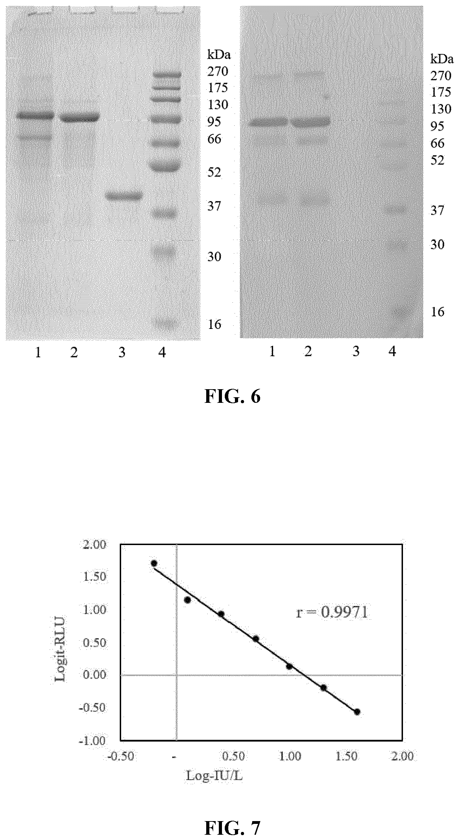

The SDS-PAGE and WB identification results were shown in FIG. 6. The band of the target protein in the expression supernatant was clearly visible. The purity of the target protein after purification could reach more than 90%, indicating that the protein soluble expression level was high, that was, the yield was high, and the cell supernatant after expression was allowed to run on the gel directly, and the relatively obvious target protein band could be seen without concentration.

### Example 2: Performance evaluation of recombinant thyroid-stimulating hormone receptor protein

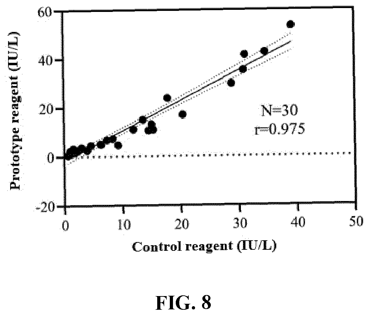

A prototype reagent for TRAb detection was constructed with the target protein, based on the acridinium ester chemiluminescent double-antibody sandwich method as mentioned in Example 1 combined with the competition method, and the obtained TRAb reagent was placed on a fully automatic chemiluminescence instrument Caris200 and the sample detection was performed according to the standard operating procedures for the fully automatic chemiluminescence instrument Caris200. First, a series of samples containing gradiently diluted TRAb were provided, and the TRAb gradient dilution samples were detected strictly in accordance with the standard operating procedures of the fully automatic chemiluminescence instrument Caris200. The detected relative luminescence intensity (RLU) and the corresponding concentration were fitted in an appropriate manner (the data were shown in Table 2), and the results showed that the correlation coefficient r of linear fitting in the range of 1 to 40 IU/L was 0.9972 (FIG. 7), indicating that this reagent showed a good linear relationship in the range of 1 to 40 IU/L. Roche TRAb electrochemiluminescence detection reagent, a mainstream commercial reagent in the market, was selected as the control reagent, and by strictly following the instructions of the commercial reagent, 30 clinical samples were detected by both the TRAb prototype detection reagent and the commercial TRAb detection reagent in parallel, and the correlation coefficient r value of the detection results of the two was 0.975 (as shown in FIG. 8), which indicated that the quantitative correlation between this reagent and the control reagent was good within the detection range, proving that the recombinant protein of the invention could be effectively used in the development of TRAb detection reagents and applied in industrial production. In summary, the above experimental results indicated that the purified protein had high activity.

**Table 2**

| Concentration, IU/L | RLU |
|---|---|
| 0.00 | 812326 |
| 0.63 | 796432 |
| 1.25 | 758456 |
| 2.50 | 727718 |
| 5.00 | 633856 |
| 10.00 | 463529 |
| 20.00 | 312326 |
| 40.00 | 171960 |

### Example 3: Stability evaluation of recombinant thyroid-stimulating hormone receptor protein:

The purified recombinant protein was subpackaged, and tested for repeated freeze-thaw activity after being frozen at -20°C. The respective signal values were detected before freezing, after one freeze-thaw cycle, two freeze-thaw cycles, three freeze-thaw cycles, four freeze-thaw cycles, and five freeze-thaw cycles. The detection was performed in duplicate, and the average value was used for analysis. The average signal value before freezing was used as the standard, and the activity changes corresponding to the signal value deviations of the samples after 1 to 5 freeze-thaw cycles were calculated for the evaluation of the freeze-thaw stability of the recombinant protein. The specific detection method was as follows: After the recombinant hTSHR antigen to be evaluated was diluted to a certain concentration, it was added to 10F8-coated magnetic particles and reacted for 15 minutes to form a 10F8-coated magnetic particle-recombinant hTSHR complex. After magnetic separation and washing, other materials that were not bound to the magnetic particles were removed. Next, acridinium ester-labeled stimulatory TRAb was added and reacted with the recombinant hTSHR in the sample for 10 minutes to form a "10F8-coated magnetic particle-recombinant hTSHR-acridinium ester-labeled stimulatory TRAb" complex. After magnetic separation and washing, other substances that were not bound to the magnetic particles were removed. Finally, after adding the pre-trigger solution and the trigger solution, the complex could emit a light signal with an intensity that could be detected by a luminometer, expressed as relative luminescence value (RLU). The results were shown in Table 3, in which even if the recombinant protein prepared by this method underwent 5 times freeze-thaw, the detection activity deviation could be controlled within 10%, that was, the freeze-thaw did not significantly affect the activity of the protein, indicating that the recombinant antigen prepared by this method had good freeze-thaw stability and could further support industrial production.

**Table 3**

| Number of freeze-thaw cycles | Activity deviation |
|---|---|
| 1 | 5% |
| 2 | 6% |
| 3 | 9% |
| 4 | 4% |
| 5 | -4% |

The above descriptions are only preferred embodiments of the present invention and are not intended to limit the present invention. For those skilled in the art, the present invention may have various modifications and changes. Any modifications, equivalent substitutions, improvements, etc. made within the spirit and principles of the present invention shall fall within the protection scope of the present invention.

## Claims

1. A recombinant thyroid-stimulating hormone receptor protein, comprising an extracellular segment of TSHR protein and an intracellular segment of TSHR protein, wherein the C-terminus of the extracellular segment of TSHR protein is connected to the N-terminus of the intracellular segment of TSHR protein.

2. The recombinant thyroid-stimulating hormone receptor protein according to claim 1, wherein the amino acid sequence of the extracellular segment of TSHR protein is set forth in SEQ ID NO: 12, and the amino acid sequence of the intracellular segment of TSHR protein is set forth in SEQ ID NO: 13.

3. The recombinant thyroid-stimulating hormone receptor protein according to claim 1, wherein the N-terminus thereof is connected to an exogenous secretion signal peptide.

4. The recombinant thyroid-stimulating hormone receptor protein according to claim 1, wherein the C-terminus of the intracellular segment of TSHR protein is further connected to a purification tag sequence.

5. A method for preparing the recombinant thyroid-stimulating hormone receptor protein according to any one of claims 1 to 4, comprising the following steps:
S1, constructing a recombinant plasmid, in which the recombinant plasmid comprises a gene fragment of the extracellular segment of TSHR protein and a gene fragment of the intracellular segment of TSHR protein; the C-terminus of the extracellular segment of TSHR protein is connected to the N-terminus of the intracellular segment of TSHR protein;
S2, transferring the recombinant plasmid into an eukaryotic expression system for expression and identification of a target protein;
S3, purifying the target protein.

6. The method according to claim 5, wherein the method for constructing the recombinant plasmid comprises: using an overlapping PCR method to ligate the gene fragment of extracellular segment of TSHR protein and the gene fragment of the intracellular segment of TSHR protein to form a target gene fragment, and then using a Gibson cloning method to construct the target gene fragment into a plasmid vector.

7. The method according to claim 5, wherein the method for constructing the recombinant plasmid comprises: chemically synthesizing a target gene fragment comprising the gene fragment of the extracellular segment of TSHR protein and the gene fragment of the intracellular segment of TSHR protein, and then constructing the target gene fragment into a plasmid vector.

8. The method according to claim 5, wherein the eukaryotic expression system is selected from the group consisting of baculovirus-insect expression system, CHO cell expression system, 293 cell expression system and yeast expression system.

9. Use of the recombinant thyroid-stimulating hormone receptor protein according to any one of claims 1 to 4 in the detection of thyroid-stimulating hormone receptor antibody.

10. A thyroid-stimulating hormone receptor antibody detection kit, comprising the recombinant thyroid-stimulating hormone receptor protein according to any one of claims 1 to 4.
